# EUROPÄISCHE PATENTSCHRIFT

(11) **EP 1 632 255 B1**
(45) Veröffentlichungstag und Bekanntmachung des Hinweises auf die Patenterteilung: **28.12.2011**
(21) Anmeldenummer: 05107594.3
(22) Anmeldetag: 18.08.2005
(51) Int. Cl.: A61L 31/02, A61L 31/14

(54) **Endoprothese aus einer Magnesiumlegierung**
Endoprosthesis made of a magnesium alloy
Endoprothèse à base d'un alliage de magnésium

(30) Priorität: 07.09.2004 DE 102004043232
(43) Veröffentlichungstag der Anmeldung: 08.03.2006
(73) Patentinhaber: Biotronik VI Patent AG, 6340 Baar (CH)
(72) Erfinder: Gerold, Bodo, 91225 Zellingen (DE); Harder, Claus, 91080, Uttenreuth (DE); Kuttler, Marc, 12205, Berlin (DE); Müller, Heinz, 91054, Erlangen (DE)
(74) Vertreter: Lindner-Vogt, Karin L.

(56) Entgegenhaltungen:
- EP-A- 1 419 793
- DE-A1- 10 128 100
- STAIGER MP ET AL: "Magnesium and its alloys as orthopedic biomaterials: a review" BIOMATERIALS, Bd. 27, 24. Oktober 2005 (2005-10-24), Seiten 1728-1734, XP002469482
- MANI G ET AL: "Coronary stents: a materials perspective" BIOMATERIALS, Bd. 28, 22. Dezember 2006 (2006-12-22), Seiten 1689-1710, XP002469483

## Beschreibung

Die Erfindung betrifft eine Endoprothese, insbesondere eine intraluminale Endoprothese wie einen Stent, mit einer Tragstruktur, die ganz oder in Teilen aus einer Magnesiumlegierung besteht.

### Stand der Technik

Viele Endoprothesen haben den Zweck, im Inneren des Körpers eines Patienten eine Stützfunktion zu übernehmen. Dementsprechend sind Endoprothesen implantierbar ausgeführt und besitzen eine Tragstruktur, die die Stützfunktion gewährleistet. Bekannt sind Implantate aus metallischen Werkstoffen. Die Wahl von Metallen als Werkstoff für die Tragstruktur eines derartigen Implantats beruht vor allem auf den mechanischen Eigenschaften von Metallen.

Metallische Stents sind in großer Zahl bekannt. Einer der Hauptanwendungsbereiche solcher Stents ist das dauerhafte Weiten und Offenhalten von Gefäßverengungen, insbesondere von Verengungen (Stenosen) der Herzkranzgefäße. Daneben sind auch Aneurysmenstents bekannt, die eine Stützfunktion für eine beschädigte Gefäßwand bieten. Derartige Stents besitzen in der Regel eine Umfangswandung von ausreichender Tragkraft, um das verengte Gefäß im gewünschten Maße offen zu halten. Um einen ungehinderten Blutfluss durch den Stent zu ermöglichen, ist diese an beiden Stirnseiten offen. Kompliziertere Ausführungen ermöglichen auch einen ungehinderten Blutfluss in Nebengefäße (Side Branch Access). Die tragende Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. An den Stent wird daher grundsätzlich die Anforderung gestellt, dass seine Tragstruktur im aufgeweiteten Zustand eine ausreichende Tragkraft aufweist, um das Gefäß offen zu halten. Um unnötige Gefäßbeschädigungen zu vermeiden, ist außerdem gewünscht, dass der Stent nach dem Aufweiten und nach Entfernen des Ballons nur wenig elastisch zurückfedert (Recoil), so dass der Stent beim Aufweiten nur wenig über den gewünschten Enddurchmesser hinaus geweitet werden muss. Weitere Kriterien, die in Bezug auf einen Stent wünschenswert sind, umfassen beispielsweise eine gleichmäßige Flächenabdeckung und eine Struktur, die eine gewisse Flexibilität in Bezug auf die Längsachse des Stents erlaubt.

In einigen Fällen, insbesondere im Falle solcher intraluminaler Endoprothesen wie Stents, ist eine dauerhafte Stützfunktion durch die Endoprothese nicht erforderlich. Vielmehr kann sich in einigen dieser Anwendungsfälle das Körpergewebe unter Anwesenheit der Stützprothese derart erholen, dass eine dauerhafte Stützwirkung durch die Prothese nicht erforderlich ist. Dies hat zu dem Gedanken geführt, solche Prothesen aus bioresorbierbarem Material zu fertigen.

Neben den gewünschten mechanischen Eigenschaften eines Stents soll dieser möglichst in einer Weise mit dem Körpergewebe am Implantationsort interagieren, dass es nicht zu erneuten Gefäßverengungen, insbesondere durch den Stent selbst hervorgerufenen Gefäßverengungen kommt. Eine Restenose (Wiederverengung des Gefäßes) soll möglichst vermieden werden. Auch ist es wünschenswert, wenn von dem Stent möglichst gar keine oder nur eine sehr geringe inflammatorische Wirkung ausgeht. In Bezug auf einen biodegradierbaren Metallstent ist es darüber hinaus wünschenswert, wenn von den Abbauprodukten des Metallstents möglichst keine oder nur sehr geringe negative physiologische Wirkungen ausgehen, bzw. sogar positive physiologische Wirkungen bedingt sind.

Aus der DE 197 31 021 ist ein bioresorbierbarer Metallstent bekannt, dessen Werkstoff als Hauptbestandteil Magnesium, Eisen oder Zink enthält. Aufgrund der mechanischen Eigenschaften, dem Degradationverhalten und der Biokompatibilität sind insbesondere Magnesiumlegierungen zu präferieren.

In DE 102 53 634 (entspricht der EP 1 419 793), DE 101 28 100 oder EP 1 395 297 wird auf den Einsatz von solchen biodegradierbaren Magnesiumlegierungen fokussiert. Die in letzteren beiden Schriften vorgeschlagenen Magnesiumlegierungen enthalten Aluminium. Das Aluminium wird dabei u.a. für die Ausbildung von Deckschichten benötigt, die die Diffusion des Magnesiums und damit den Degradationsprozess verlangsamen sollen. Dies ist gemäß diesen Schriften erforderlich, um eine ausreichend lange mechanische Stabilität der Endoprothese zu erreichen und Gasausscheidungen beim Degradationsprozess zu verhindern / zu mindern.

Aluminium ist aber dafür bekannt gesundheitliche Schäden zu verursachen, insbesondere wenn es in ionischer Form vorliegt. So ist Aluminium u.a. dafür bekannt, Schäden am Zentralnervensystem zu verursachen und Symptome wie Demenz, Gedächtnisverlust, Antriebslosigkeit oder heftiges Zittern auszulösen. Aluminium gilt als Risikofaktor für die Alzheimer-Krankheit (Harold D. Foster Ph. D., Journal für Orthomolekulare Medizin 2/01).

Auch negative Effekte hinsichtlich der Biokompatibilität in unmittelbarer Umgebung von Endoprothesen aus Al-haltigen Magnesiumlegierungen konnten in Experimenten beobachtet werden. So wurden in Tierexperimenten pathologische Höfe um die degradierenden Stege solcher Stents sowie eine ausgeprägte Neointimahyperplasie beobachtet, die dem eigentlichen Zweck des Stents, den Gefäßverschluss zu verhindern, entgegenwirkt. Der Einsatz von Aluminium in Endoprothesen, insbesondere Stents, ist somit ungünstig.

Eine Reizung des umliegenden Gewebes, die meist mit Entzündungen und Neointimahyperplasie einhergeht und Restenose zur Folge hat, wird abgesehen von fehlender Biokompatibilität der verwendeten Materialien (z.B. Al) auch durch die mechanische Reizung des Implantats ausgelöst. Bisherige Ansätze die mechanische Reizung durch das Implantat zu reduzieren, beruhen auf der Reduzierung der Auflageflächen des Implantates, welche die Reizung erzeugt. Solche Verfahren haben aber Grenzen, bedingt durch die benötigten mechanischen Eigenschaften, sowie die ab einer kritischen Breite auftretende Klingenwirkung.

Generell vernachlässigt wurde bislang der Ansatz, körpereigene Heilungsprozesse im Rahmen der Anwendung von Endoprothesen zu aktivieren, um so den Heilungsprozess weiter zu verbessern.

Vor diesem Hintergrund liegt der vorliegenden Erfindung die Aufgabe zugrunde, eine biodegradierbare Endoprothese auf der Basis einer Magnesiumlegierung bereit zu stellen, die die geschilderten Nachteile des Standes der Technik vermeidet. Insbesondere sollen Degradations- und Biokompatibilitätseigenschaften optimiert und/oder körpereigene Heilungsprozesse aktiviert, gesteigert und unterstützt werden.

Erfindungsgemäß wird diese Aufgabe durch eine Endoprothese nach Anspruch 1 gelöst. Die Endoprothese weist eine Tragstruktur auf, die ganz oder in Teilen aus einer Magnesiumlegierung folgender Zusammensetzung besteht:
- Seltenerdmetalle 2.0 bis 5.0 Gew.%, dabei Neodym 2.0 bis 2.5 Gew.%, wobei die Sammelbezeichnung "Seltenerdmetalle" die Elemente Scandium (Ordnungszahl 21), Lanthan (57) sowie die Lanthanoide (58 bis 71) umfasst,
- Yttrium 3.5 bis 4.5 Gew.%,
- Zirkonium 0.3 bis 1.0 Gew.%,
- Rest 0 bis 0.5 Gew.%,
wobei Magnesium den auf 100 Gew.% verbleibenden Gewichtsanteil an der Legierung einnimmt. Die Legierung zeigt sehr günstige mechanische und physiologische Eigenschaften und ein günstiges Degradationsverhalten in vivo. Sie kann einfach verarbeitet werden und zeigt in ersten Studien einen positiven physiologischen Effekt auf das umgebene Gewebe in Mensch und Tier, wenn die Legierung in Endoprothesen, insbesondere Stents, verwendet wird.

Die Sammelbezeichnung ,Seltenerdmetall' steht für die Elemente Scandium (Ordnungszahl 21), Lanthan (57) sowie die 14 auf das Lanthan folgenden Elemente Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), die als Lanthanoide bezeichnet werden. Der Anteil der Seltenerdmetalle an der Magnesiumlegierung umfasst somit auch den Anteil des Neodyms. Letzterer Anteil ist ebenfalls auf das Gesamtgewicht der Legierung bezogen und muss im angegebenen Bereich liegen. Liegt der Anteil an Neodym in der Legierung z.B. bei 2.0 Gew.% und der Anteil an Seltenerdmetallen bei 2.5 Gew.%, so werden zwangsläufig Seltenerdmetalle außer Neodym einen Gewichtsanteil an der Legierung von 0.5 Gew.% aufweisen.

Der Rest enthält vorzugsweise nur die aus dem Herstellungsprozess der Magnesiumlegierung bedingten Verunreinigungen. Mit anderen Worten, es sind lediglich spezifische Verunreinigungen enthalten, die sich bei der Herstellung der Legierung nicht vermeiden lassen oder Restkomponenten, die bewusst der Legierung zugesetzt werden. Hierdurch werden die positiven physiologischen Effekte und die mechanischen Eigenschaften des Werkstoffs sichergestellt und teils gar erst erreicht.

Ergänzend oder alternativ zu vorgenannter bevorzugter Variante enthält der Rest kein oder allenfalls < 0.01 Gew.% Aluminium. Gerade Aluminium hat einen ausgeprägten negativen Einfluss auf das physiologische Verhalten, wie Materialuntersuchungen sowie in vivo und in vitro Untersuchungen gezeigt haben.

Aufgrund der negativen Eigenschaften, insbesondere auf die Biokompatibilität, werden in den Legierungen neben dem Element Aluminium (Al) vorzugsweise auch die Elemente Kupfer (Cu), Nickel (Ni), Silber (Ag), Quecksilber (Hg), Cadmium (Cd), Beryllium (Be) oder Chrom (Cr) vermieden, d.h. die Elemente sind abgesehen von herstellungsbedingten Verunreinigungen nicht in der Legierung enthalten. Der als Rest bezeichnete Anteil der Legierung beinhaltet vorrangig Massenanteile eines, mehrerer oder aller genannter Elemente unter den folgenden Grenzen:
- Aluminium < 0.01 Gew.%,
- Kupfer < 0.03 Gew.%,
- Nickel < 0.005 Gew.%,
- Silber < 0.01 Gew.%,
- Quecksilber < 0.03 Gew.%,
- Cadmium < 0.03 Gew.%,
- Beryllium < 0.03 Gew.%,
- Chrom < 0.03 Gew.%.

Die Vermeidung dieser Elemente ist für den Zweck der Erfindung von Bedeutung, da diese gesundheitsschädliche Wirkung haben, die mechanischen Eigenschaften der Legierung unerwünscht beeinflussen sowie die auf den Heilungsprozess positiven Einflüsse der Legierung und insbesondere des Magnesiums beeinträchtigen. Bekanntlich können schon geringe Spuren von Verunreinigungen einen metallurgisch und/oder physiologisch erheblichen Effekt haben. Das Identifizieren der Störelemente und insbesondere das Festlegen von Grenzwerten für diese Störelemente liefert damit einen erheblichen technischen Beitrag zur Optimierung der Produkte.

Bevorzug ist hingegen, dass der Rest die Elemente Lithium und/oder Zink enthält. Der Anteil der Komponenten an der Legierung liegt vorzugsweise bei 0.1 bis 0.5 Gew.%, wobei im Falle des Zusatzes von Lithium und Zink deren kumulierter Gesamtanteil maximal 0.5 Gew.% beträgt. Die Anwesenheit dieser Elemente hat offenbar positiven Einfluss auf die mechanischen Eigenschaften und die Biokompatibilität des Implantats.

Mit der hier beschriebenen Magnesiumlegierung konnte ein signifikant verbesserter Degradationsablauf mit deutlich besserer Reproduzierbarkeit erzielt werden, als bisher z.B. für aluminiumhaltige Magnesiumlegierungen bekannt ist (Heart (2003) 89, 651-656). Insbesondere die Reproduzierbarkeit des Degradationsverlaufes ist für eine medizinische Anwendung unabdingbar. Dank des verwirklichten kontrollierten und langsamen Degradationsprozesses entstehen keine oder allenfalls geringe Gasausscheidungen.

Es wurde in vivo und in vitro nachgewiesen, dass die Legierung und Ihre Abbauprodukte äußerst biokompatibel sind. Unter Verwendung der Magnesiumlegierung konnte starken immunologischen Reaktionen des Körpers entgegengewirkt werden. Auch eine kontrolliertes Zellwachstum, insbesondere humaner glatter Muskelzellen und Endothelzellen, konnte anhand von in vitro Versuchen belegt werden. Unkontrollierte Zellwucherungen, die zur Restenose führen können, scheinen verhindert bzw. stark eingedämmt zu sein. Dies ist insbesondere bei der Verwendung von aluminiumhaltigen Legierungen, bei denen starke Neointimahyperplasie beobachtet wurde, derart nicht der Fall. Der den positiven Effekten zugrunde liegende Wirkungsmechanismus ist bisher im Detail nicht ergründet.

Einen Beitrag zur besonderen Verträglichkeit des Implantats könnte Magnesium liefern. Allgemein bekannte Wirkungen und Einflüsse des üblicher Weise über die Ernährung aufgenommenen Magnesiums auf die Körperfunktionen lassen vermuten, dass solche Prozesse auch beim Einsatz von Magnesium als Implantat in geeigneter Legierungszusammensetzung zumindest lokal aktiviert werden.

Es ist z.B. bekannt, dass Magnesium im Organismus einen positiven Einfluss auf die Wundheilung hat, da dieses für den anaeroben Stoffwechsel notwendig ist und die normale Granulation des Bindegewebes fördert und unkontrolliertes Zellwachstum eher verhindert (Dr. med. Dr. sc. Nat PG Seeger, SA-NUM-Post Nr. 13/1990, 14-16).

Ein weiterer positiver Aspekt bei der Verwendung von Magnesium ist, dass die unspezifische Abwehr über das Properdinsystem nur bei Anwesenheit von Magnesium wirksam ist und die Phagozytose von Bakterien durch Leukozyten eine Anregung durch das Magnesium erfährt. Somit sorgt Magnesium u.a. für die Bekämpfung von Infektionen durch Unterstützung bzw. Aktivierung des körpereigenen Immunsystems und mindert die Anfälligkeit für Infektionen. Unerwünschten infektionsbedingten Entzündungserscheinungen aufgrund von Kontaminationen, die im Rahmen der Anwendung einer Endoprothese auftreten können, und die wiederum Auslöser für eine Restenose sein können, wird somit entgegengewirkt.

Die hier verwendete Legierung wirkt auch positiv gegen mechanisch induzierte Restenose. Erreicht wird dies zum einen durch die mechanischen Eigenschaften der verwendeten Legierung, die sich durch ein günstiges Elastizitätsmodul auszeichnen. Des Weiteren wird die allgemein bekannte muskelrelaxierende Wirkung des Magnesium (Ca-Antagonist) genutzt, um mechanische Reizungen zu vermindern. Es ist zu erwarten, dass das Magnesium in der Legierung bzw. die magnesiumhaltigen Zerfallsprodukte bei der Degradation eine Relaxierung der in der näheren Umgebung vorhandenen Muskelzellen fördern. Dies ist insbesondere bei Stent-Anwendungen vorteilhaft, da nicht nur die mechanische Reizung reduziert wird, sondern auch das Gefäß durch das lokal relaxierte Muskelgewebe zusätzlich offen gehalten werden kann.

Ferner ist bevorzugt, dass - unabhängig voneinander - der Anteil an Yttrium 3.8 Gew.% bis 4.5 Gew.% und der Anteil der Seltenerdmetalle 2.8 Gew.% bis 3.4 Gew.% beträgt. Hierdurch lässt sich die physiologische Verträglichkeit der Legierung und seiner Abbauprodukte noch steigern sowie das Degradationsverhalten für die geplanten Zwecke optimieren.

Die Magnesiumlegierung wird vorzugsweise stranggepresst. Es hat sich herausgestellt, dass die Verarbeitung der Legierung dessen physiologische Wirkung beeinflusst. Diese physiologischen Eigenschaften sind somit zumindest zum Teil durch den Herstellungsprozess bedingt.

Die Endoprothese ist vorzugsweise als intraluminale Endoprothese ausgebildet. Besonders bevorzugt ist eine Endoprothese, die als Stent, insbesondere als Koronarstent oder als peripherer Stent ausgebildet ist. Aufgrund der positiven Eigenschaften der genannten Magnesiumlegierung besteht die Tragstruktur der Endoprothese vorzugsweise zur Gänze aus der Magnesiumlegierung.

Nach einer bevorzugten Variante für die Anwendung der Legierung als Stent, insbesondere als Koronarstent oder als peripherer Stent, wird die spezifische Zusammensetzung der Magnesiumlegierung sowie deren Modifikation durch die Herstellungsweise und das Stent-Design dahingehend vorgegeben, dass die Zersetzung unmittelbar nach der Implantation einsetzt und die mechanische Integrität für mindestens 5 Tage bis höchstens 1 Jahr Aufrecht erhalten wird. Unter "mechanischer Integrität" wird dabei die trotz fortschreitendem Abbau noch ausreichende Stabilität der Strukturelemente des Implantates verstanden, die zur Erfüllung des medizinischen Zwecks des Implantats, d.h. die Aufrechterhaltung der erforderlichen Stützfunktion dienen. Besonders bevorzugt beträgt der Zeitraum 10 bis 90 Tage.

Nachfolgend wird die Erfindung anhand eines Ausführungsbeispiels und dazu gehöriger Zeichnungen näher erläutert. Es zeigen:
Figur 1 eine schematische Darstellung einer Endoprothese in Form eines Stents und
Figur 2 eine Abwicklung der Tragstruktur des Stents aus Figur 1.

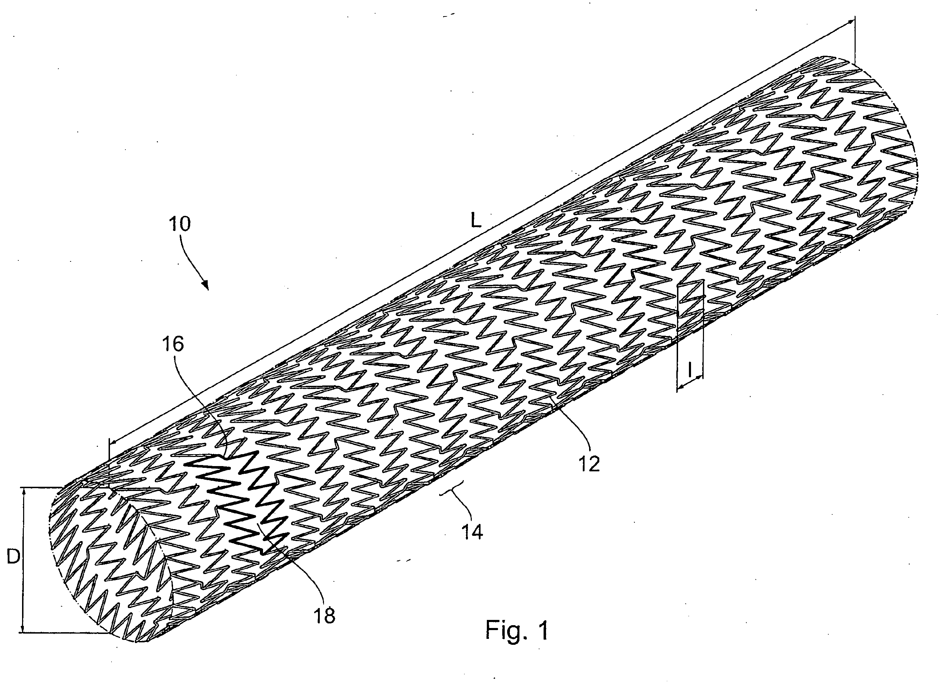

Figur 1 zeigt eine Endoprothese als endoluminale Prothese in Form eines Stents 10 mit einer Tragstruktur. Dieser Stent 10 und seine Tragstruktur haben die Form eines an seinen Stirnseiten offenen Hohlköpers, dessen Umfangswandung von der Tragstruktur gebildet ist, die wiederum von teilweise gefalteten Stegen 12 gebildet ist. Die Stege 12 bilden Stützabschnitte 14, welche von jeweils einem in Längsrichtung ringförmig geschlossenen, zick-zack- oder mäanderförmig gefalteten Steg 12 gebildet sind. Der Stent 10 eignet sich für den koronaren Einsatz.

Die Tragstruktur des Stents 10 wird von mehreren solcher in Längsrichtung aufeinanderfolgender Stützabschnitte 12 gebildet. Die Stützabschnitte 12 bzw. Stegringe 14 sind über Verbindungsstege 16 miteinander verbunden. Jeweils zwei in Umfangsrichtung aneinander benachbarte Verbindungsstege 16 sowie die zwischen diesen Verbindungsstegen 16 einander gegenüberliegenden Teilabschnitte der Stegringe 14 oder Stützabschnitte 12 definieren eine Masche 18 des Stents 10. Eine solche Masche 18 ist in Figur 1 hervorgehoben dargestellt. Jede Masche 18 umschließt eine radiale Öffnung der Umfangswandung bzw. der Tragstruktur des Stents 10.

Jeder Stegring 14 weist etwas drei bis sechs über den Umfang des Stents 10 gleich verteilte Verbindungsstege 16 auf, die jeweils einen Stegring 14 mit dem benachbarten Stegring 14 verbinden. Dementsprechend weist der Stent 10 in Umfangsrichtung zwischen zwei Stützabschnitten 14 jeweils drei bis sechs Maschen 18 auf.

Aufgrund der Faltung der Stege 12 ist der Stent 10 in Umfangsrichtung expandierbar. Dies geschieht beispielsweise mit einem an sich bekannten Ballonkatheter, der an seinem distalen Ende einen mittels eines Fluids expandierbaren Ballon aufweist. Der Stent 10 ist im komprimierten Zustand auf den deflatierten Ballon aufgecrimpt. Mit Expansion des Ballons werden sowohl der Ballon als auch der Stent 10 aufgeweitet. Anschließend kann der Ballon wieder deflatiert werden und der Stent 10 löst sich von dem Ballon. Auf diese Weise kann der Katheter gleichzeitig dem Einführen des Stents 10 in ein Blutgefäß und insbesondere in ein verengtes Herzkranzgefäß sowie zum Expandieren des Stents 10 an diesem Ort dienen.

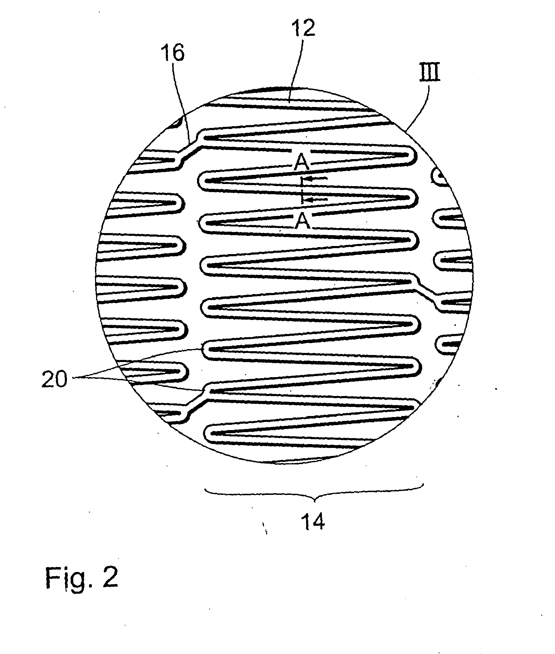

In Figur 2 ist ein Ausschnitt aus einer Abwicklung der Umfangswandung des Stents 10 dargestellt. Die Abwicklung zeigt den komprimierten Zustand des Stents 10.

Die Tragstruktur des in den Figuren dargestellten Stents 10 besteht vollständig aus einer biodegradierbaren Magnesiumlegierung folgender Zusammensetzung:
Seltenerdmetalle 3.0 Gew.%, dabei Neodym 2.3 Gew.%,
Yttrium 4.0 Gew.%,
Zirkonium 0.5 Gew.%,
Lithium 0.4 Gew.%,
Aluminium, Silber, Kupfer, Quecksilber, Cadmium, Beryllium, Chrom und Nickel < 0.005 Gew.%*, (*Nachweisgrenze bei der Bestimmung)
wobei Magnesium den auf 100 Gew.% verbleibenden Gewichtsanteil an der Legierung einnimmt.

## Patentansprüche

1. Endoprothese mit einer Tragstruktur, die ganz oder in Teilen aus einer Magnesiumlegierung folgender Zusammensetzung besteht:
- Seltenerdmetalle 2.0 bis 5.0 Gew.%, dabei Neodym 2.0 bis 2.5 Gew.%, wobei die Sammelbezeichnung "Seltenerdmetalle" die Elemente Scandium (Ordnungszahl 21), Lanthan (57) sowie die Lanthanoide (58 bis 71) umfasst,
- Yttrium 3.5 bis 4.5 Gew.%,
- Zirkonium 0.3 bis 1.0 Gew.%,
- Rest 0 bis 0.5 Gew.%,
wobei Magnesium den auf 100 Gew.% verbleibenden Gewichtsanteil an der Legierung einnimmt.

2. Endoprothese nach Anspruch 1, **dadurch gekennzeichnet, dass** der Anteil an Yttrium 3.8 Gew.% bis 4.5 Gew.% beträgt.

3. Endoprothese nach Anspruch 1, **dadurch gekennzeichnet, dass** der Anteil der Seltenerdmetalle 2.8 Gew.% bis 3.4 Gew.% beträgt.

4. Endoprothese nach Anspruch 1, **dadurch gekennzeichnet, dass** der Rest nur die aus dem Herstellungsprozess der Magnesiumlegierung bedingten Verunreinigungen enthält.

5. Endoprothese nach Anspruch 1, **dadurch gekennzeichnet, dass** der Rest kein oder allenfalls < 0.01 Gew.% Aluminium enthält.

6. Endoprothese nach Anspruch 1, **dadurch gekennzeichnet, dass** der Rest kein oder allenfalls < 0.03 Gew.% Kupfer enthält.

7. Endoprothese nach Anspruch 1, **dadurch gekennzeichnet, dass** der Rest kein oder allenfalls < 0.005 Gew.% Nickel enthält.

8. Endoprothese nach Anspruch 1, **dadurch gekennzeichnet, dass** der Rest kein oder allenfalls < 0.01 Gew.% Silber enthält.

9. Endoprothese nach Anspruch 1, **dadurch gekennzeichnet, dass** der Rest kein oder allenfalls < 0.03 Gew.% Quecksilber enthält.

10. Endoprothese nach Anspruch 1, **dadurch gekennzeichnet, dass** der Rest kein oder allenfalls < 0.03 Gew.% Cadmium enthält.

11. Endoprothese nach Anspruch 1, **dadurch gekennzeichnet, dass** der Rest kein oder allenfalls < 0.03 Gew.% Chrom enthält.

12. Endoprothese nach Anspruch 1, **dadurch gekennzeichnet, dass** der Rest kein oder allenfalls < 0.03 Gew.% Beryllium enthält.

13. Endoprothese nach Anspruch 1, **dadurch gekennzeichnet, dass** der Rest Lithium und/oder Zink enthält.

14. Endoprothese nach einem der vorhergehenden Ansprüche, **dadurch gekennzeichnet, dass** die Tragstruktur stranggepresst ist.

15. Endoprothese nach einem der vorhergehenden Ansprüche, **dadurch gekennzeichnet, dass** die Endoprothese als intraluminale Endoprothese ausgebildet ist.

16. Endoprothese nach Anspruch 15, **dadurch gekennzeichnet, dass** die Endoprothese als Stent ausgebildet ist.

17. Endoprothese nach Anspruch 16, **dadurch gekennzeichnet, dass** die Endoprothese als Koronarstent oder als peripherer Stent ausgebildet ist.

18. Endoprothese nach den Anspruch 17, **dadurch gekennzeichnet, dass** eine spezifische Zusammensetzung der Magnesiumlegierung sowie deren Modifikation dahingehend vorgegeben sind, dass eine Zersetzung unmittelbar nach der Implantation einsetzt und die mechanische Integrität für mindestens 5 Tage bis höchstens 1 Jahr Aufrecht erhalten wird.

## Claims

1. An endoprosthesis comprising a support structure composed in entirety or in parts of a magnesium alloy having the following composition:
- Rare-earth metals 2.0 to 5.0% by weight, including neodymium 2.0 to 2.5% by weight, wherein the umbrella term "rare-earth metals" includes the elements scandium (atomic number 21), lanthanum (57) and the lanthanoids (58 to 71),
- Yttrium 3.5 to 4.5% by weight,
- Zirconium 0.3 to 1.0% by weight,
- The remainder 0 to 0.5% by weight,
wherein magnesium makes up the portion by weight of the alloy remaining to make 100% by weight.

2. The endoprosthesis according to claim 1, **characterized in that** the portion of yttrium is 3.8% by weight to 4.5% by weight.

3. The endoprosthesis according to claim 1, **characterized in that** the portion of rare-earth metals is 2.8% by weight to 3.4% by weight.

4. The endoprosthesis according to claim 1, **characterized in that** the remainder contains only the impurities resulting from the manufacturing process of the magnesium alloy.

5. The endoprosthesis according to claim 1, **characterized in that** the remainder contains no aluminum or, at most, < 0.01 % by weight of aluminum.

6. The endoprosthesis according to claim 1, **characterized in that** the remainder contains no copper or, at most, < 0.03% by weight of copper.

7. The endoprosthesis according to claim 1, **characterized in that** the remainder contains no nickel or, at most, < 0.005% by weight of nickel.

8. The endoprosthesis according to claim 1, **characterized in that** the remainder contains no silver or, at most, < 0.01 % by weight of silver.

9. The endoprosthesis according to claim 1, **characterized in that** the remainder contains no mercury or, at most, < 0.03% by weight of mercury.

10. The endoprosthesis according to claim 1, **characterized in that** the remainder contains no cadmium or, at most, < 0.03% by weight of cadmium.

11. The endoprosthesis according to claim 1, **characterized in that** the remainder contains no chromium or, at most, < 0.03% by weight of chromium.

12. The endoprosthesis according to claim 1, **characterized in that** the remainder contains no beryllium or, at most, < 0.03% by weight of beryllium.

13. The endoprosthesis according to claim 1, **characterized in that** the remainder contains lithium and/or zinc.

14. The endoprosthesis according to one of the preceding claims, **characterized in that** the support structure is extruded.

15. The endoprosthesis according to one of the preceding claims, **characterized in that** the endoprosthesis is in the form of an intraluminal endoprosthesis.

16. The endoprosthesis according to claim 15, **characterized in that** the endoprosthesis is in the form of a stent.

17. The endoprosthesis according to claim 16, **characterized in that** the endoprosthesis is in the form of a coronary stent or a peripheral stent.

18. The endoprosthesis according to claim 17, **characterized in that** a specific composition of the magnesium alloy and the modification thereof are specified such that decomposition starts immediately after implantation, and the mechanical integrity is retained for at least 5 days to up to 1 year.

## Revendications

1. Endoprothèse avec une structure de support constituée entièrement ou partiellement d'un alliage de magnésium avec la composition suivante :
- 2,0 à 2,5% en poids de métaux des terres rares, dont 2,0 à 2,5% en poids de néodyme, où le terme collectif de « métaux de terres rares » inclut le scandium (nombre de charge 21), le lanthane (57) ainsi que les lanthanides (58 à 71),
- 3,5 à 4,5% en poids d'yttrium,
- 0,3 à 1,0% en poids de zirconium,
- 0 à 0,5% de résidus,
dans laquelle le magnésium occupe la part de poids restante sur 100% en poids dans l'alliage.

2. Endoprothèse selon la revendication 1, **caractérisée en ce que** la part d'yttrium est de 3,8% en poids à 4,5% en poids.

3. Endoprothèse selon la revendication 1, **caractérisée en ce que** la part des métaux de terres rares est de 2,8% en poids à 3,4% en poids.

4. Endoprothèse selon la revendication 1, **caractérisée en ce que** les résidus ne contiennent que les impuretés issues du procédé de fabrication de l'alliage de magnésium.

5. Endoprothèse selon la revendication 1, **caractérisée en ce que** les résidus ne contiennent pas ou tout au plus < 0,01% en poids d'aluminium.

6. Endoprothèse selon la revendication 1, **caractérisée en ce que** les résidus ne contiennent pas ou tout au plus < 0,03% en poids de cuivre.

7. Endoprothèse selon la revendication 1, **caractérisée en ce que** les résidus ne contiennent pas ou tout au plus < 0,005% en poids de nickel.

8. Endoprothèse selon la revendication 1, **caractérisée en ce que** les résidus ne contiennent pas ou tout au plus < 0,01% en poids d'argent.

9. Endoprothèse selon la revendication 1, **caractérisée en ce que** les résidus ne contiennent pas ou tout au plus < 0,03% en poids de mercure.

10. Endoprothèse selon la revendication 1, **caractérisée en ce que** les résidus ne contiennent pas ou tout au plus < 0,03% en poids de cadmium.

11. Endoprothèse selon la revendication 1, **caractérisée en ce que** les résidus ne contiennent pas ou tout au plus < 0,03% en poids de chrome.

12. Endoprothèse selon la revendication 1, **caractérisée en ce que** les résidus ne contiennent pas ou tout au plus < 0,03% en poids de béryllium.

13. Endoprothèse selon la revendication 1, **caractérisée en ce que** les résidus contiennent du lithium et/ou du zinc.

14. Endoprothèse selon l'une des revendications précédentes, **caractérisée en ce que** la structure de support est extrudée.

15. Endoprothèse selon l'une des revendications précédentes, **caractérisée en ce que** l'endoprothèse est conçue comme une endoprothèse intraluminale.

16. Endoprothèse selon la revendication 15, **caractérisée en ce que** l'endoprothèse est conçue comme un stent.

17. Endoprothèse selon la revendication 16, **caractérisée en ce que** l'endoprothèse est conçue comme un stent coronaire ou comme un stent périphérique.

18. Endoprothèse selon la revendication 17, **caractérisée en ce qu'**une composition spécifique de l'alliage de magnésium ainsi que de sa modification est prescrite de manière à ce qu'une décomposition commence immédiatement après l'implantation et que l'intégrité mécanique soit maintenue pendant au moins 5 jours et tout au plus pour 1 an.
